(19) **Europäisches Patentamt / European Patent Office / Office européen des brevets**

(11) **EP 2 849 646 B1**

(12) **FASCICULE DE BREVET EUROPEEN**

(45) Date de publication et mention
de la délivrance du brevet:
**26.07.2017 Bulletin 2017/30**

(21) Numéro de dépôt: **13723092.6**

(22) Date de dépôt: **14.05.2013**

(51) Int Cl.:
***A61B 5/113*** (2006.01)

(86) Numéro de dépôt international:
**PCT/EP2013/059863**

(87) Numéro de publication internationale:
**WO 2013/171178 (21.11.2013 Gazette 2013/47)**

(54) **DISPOSITIF ET MÉTHODE DE DÉTECTION DE MOUVEMENTS RESPIRATOIRES**

VORRICHTUNG UND VERFAHREN ZUR ERKENNUNG VON ATEMBEWEGUNGEN

DEVICE AND METHOD FOR DETECTING RESPIRATORY MOVEMENTS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.05.2012 BE 201200319**

(43) Date de publication de la demande:
**25.03.2015 Bulletin 2015/13**

(73) Titulaire: **2-Observe S.A.**
**1435 Mont-Saint-Guibert (BE)**

(72) Inventeur: **HERMANNE, Jean-Philippe**
**B-4570 Vyle et Tharoul (BE)**

(74) Mandataire: **Le Quéré, Hervé Yves et al**
**Abyoo sprl**
**Centre Monnet**
**Avenue Jean Monnet, 1**
**1348 Louvain-la-Neuve (BE)**

(56) Documents cités:
**WO-A1-2005/020815    WO-A2-03/005893**
**US-A- 4 745 284    US-A- 5 309 921**
**US-A1- 2008 269 625**

EP 2 849 646 B1

## Description

### Domaine de l'invention

L'invention se rapporte à un procédé de surveillance de la respiration d'un patient, humain ou animal, par l'analyse d'ondes infrarouges reçues par au moins un détecteur à infrarouge passif. L'invention se rapporte également à un dispositif de surveillance de la respiration d'un patient, humain ou animal.

### État de la technique

Depuis de nombreuses années, des recherches sont effectuées dans le domaine des dispositifs et des procédés de détection de troubles respiratoires notamment lors du sommeil, tels que la bradypnée, l'hypopnée ou l'apnée. Ainsi, un grand nombre de dispositifs ont été développés afin de détecter des troubles respiratoires, de surveiller le rythme respiratoire d'un individu ou d'un animal sur une période de temps plus ou moins longue. Cependant, les dispositifs et les méthodes actuelles présentent un certain nombre d'inconvénients. Certains sont invasifs, c'est-à-dire qu'ils nécessitent un contact physique avec l'individu ou l'animal, ce qui peut présenter une certaine gêne pour l'individu ou l'animal voire être incompatible avec la présence d'autres dispositifs utilisés en milieu hospitalier ou vétérinaire. Par exemple, l'appareil de polysomnographie décrit dans le document US20100331632 doit être ceinturé autour de l'individu ou de l'animal afin de permettre la détection de troubles respiratoires. D'autres dispositifs ne nécessitent pas de contact physique, mais ces appareils sont soit onéreux soit ce sont des dispositifs qui émettent des ondes, comme par exemple des ondes électromagnétiques, dont on ne peut négliger un rôle potentiellement pathogène sur le corps humain ou animal, sans négliger d'éventuelles perturbations d'autres appareils médicaux lors de l'utilisation de ce type d'appareil dans un cadre hospitalier ou vétérinaire.

Le document WO2005020815 décrit l'utilisation de détecteurs à infrarouge passifs dans le cadre d'une méthode de surveillance du rythme respiratoire d'un individu ou d'un animal. Des détecteurs à infrarouge passifs à haute sensibilité sont disposés autour de l'individu ou de l'animal. Ces appareils détectent les mouvements de la cage thoracique, et transmettent les données liées à ces mouvements à un amplificateur via un contrôleur logique. L'amplificateur est lui-même relié à une alarme. Lorsqu'aucun des détecteurs ne détecte de mouvement durant une certaine période de temps, cette alarme est déclenchée, permettant de réveiller l'individu, ou de prévenir un autre individu de cet évènement.

Cependant, ce dispositif, bien qu'utile et présentant des avantages certains, n'est pas totalement satisfaisant du fait du nombre de faux positifs et de faux négatifs qu'il génère. Les détecteurs à infrarouges, du fait de leur grande sensibilité, reçoivent un important bruit de fond rendant souvent les signaux inexploitables et génèrent de fausses informations, ce qui est incompatible avec un dispositif destiné à favoriser la détection de troubles physiologiques importants, comme la présence d'un arrêt respiratoire de type apnée centrale d'étiologies multiples. Il s'avère très difficile de distinguer les signaux générés par un mouvement de la cage thoracique des signaux générés par l'environnement. La détermination de la présence ou de l'absence d'une ampliation du thorax par un simple seuil de détection arbitrairement choisi peut entrainer de nombreuses erreurs.

### Résumé de l'invention

Un des buts de la présente invention est de résoudre au moins partiellement les problèmes liés à la surveillance de la respiration d'un individu ou d'un animal. Notamment en proposant un dispositif et une méthode de détection de mouvements respiratoires ne nécessitant aucun contact avec l'individu ou l'animal, et permettant une détection de la présence ou de l'absence d'un mouvement respiratoire plus fiable.

A cette fin, le procédé de surveillance de la respiration d'un patient selon l'invention est caractérisé en ce qu'il comprend les étapes consistant à :

- fournir au moins un détecteur à infrarouge passif ciblant au moins un endroit du patient susceptible de présenter des mouvements respiratoires,
- fournir, selon une fréquence d'échantillonnage fE, au moins une suite de signaux discrets de mesure ayant comme origine l'au moins un détecteur à infrarouge passif,
- définir une série de N fenêtres temporelles glissantes, débutant à des moments successifs différents $T_{in,i}$, i étant un entier compris entre 1 et N représentant chaque fenêtre temporelle glissante, de longueur $\Delta T_i = T_{f,i} - T_{in,i}$, $T_{f,i}$ désignant un temps final d'une fenêtre temporelle glissante numéro i,
- soustraire à chacun desdits signaux discrets de mesure présents dans une même fenêtre temporelle glissante une composante continue calculée sur une pluralité de ces signaux discrets de mesure présents dans une même fenêtre temporelle glissante pour obtenir au moins une suite temporelle de signaux discrets utiles,
- générer à chaque temps final $T_{f,i}$ un indicateur de mouvements respiratoires calculé à partir d'une amplitude moyenne calculée sur un ensemble de signaux discrets utiles compris dans une fenêtre temporelle glissante de longueur $\Delta T_i$ précédant ledit temps $T_{f,i}$ de la fenêtre temporelle glissante numéro i.

Une telle méthode permet d'analyser des signaux discrets de mesure générés par au moins un détecteur à infrarouge passif ciblant au moins un endroit du patient susceptible de présenter des mouvements respiratoires, et de mieux discriminer les détections de mouvements erronées des détections réelles. Cette méthode permet également d'analyser des signaux de faible amplitude en présence d'un bruit de fond important. Ainsi, la détection d'une absence de mouvement respiratoire se fait avec une grande précision, et l'alarme ne se déclenche pas de façon inopportune.

Dans un mode de réalisation particulier de l'invention, le procédé comporte une étape de calcul d'une amplitude moyenne consistant à :

- calculer une valeur RMS d'un ensemble de signaux discrets utiles compris dans une fenêtre temporelle glissante de longueur $\Delta T_i$,
- comparer ladite valeur RMS avec un seuil prédéfini de détection d'un mouvement respiratoire par calcul d'une amplitude moyenne.

Le procédé selon ce mode de réalisation permet de comparer pour chaque fenêtre temporelle glissante l'amplitude moyenne d'un ensemble de signaux discrets utiles de ladite fenêtre temporelle considérée avec des valeurs prédéfinies, améliorant encore plus la sensibilité de détection du procédé.

Dans un autre mode de réalisation particulier de l'invention, le procédé comporte une étape d'analyse spectrale d'un ensemble de signaux discrets utiles compris dans une fenêtre temporelle glissante, suivi de la détermination des deux plus grands pics présents dans l'analyse spectrale de l'ensemble de signaux discrets utiles compris dans ladite fenêtre temporelle glissante, suivi d'une étape de comparaison entre la fréquence du plus grand pic avec une plage de fréquence déterminée, suivi du calcul d'un coefficient pour déterminer la présence ou l'absence d'un mouvement respiratoire. Grâce à ce mode de réalisation, lorsqu'un ensemble de signaux discrets utiles ne se distingue pas assez des signaux générés par un bruit de fond et transmis par l'au moins un détecteur à infrarouge passif ciblant au moins un endroit du patient susceptible de présenter des mouvements respiratoires, il est possible de classer un ensemble de signaux discrets utiles compris dans une fenêtre d'analyse glissante, améliorant encore plus la sensibilité du procédé pour détecter la présence ou l'absence d'un mouvement respiratoire.

L'invention porte également sur un dispositif de détection de la présence ou de l'absence d'un mouvement respiratoire chez un individu ou chez un animal.

Ces aspects ainsi que d'autres aspects de l'invention seront clarifiés dans la description détaillée de modes de réalisation particuliers de l'invention.

## Brève description des figures

Référence étant faite aux dessins des figures, dans lesquelles :

la Fig.1 représente de manière schématique un dispositif selon l'invention.

la Fig.2 représente de manière schématique un mode de réalisation particulier d'un dispositif selon l'invention.

la Fig.3 représente de manière schématique différentes étapes d'un procédé de réalisation de l'invention.

la Fig.4 représente de manière schématique des étapes d'un mode de réalisation particulier du procédé selon l'invention.

La Fig.5a représente de manière schématique une superposition de fenêtres temporelles glissantes de longueur $\Delta T_i$ séparées par un pas de temps constant.

La Fig.5b représente de manière schématique certaines étapes d'un procédé de réalisation de l'invention.

Les dessins des figures ne sont pas à l'échelle. Généralement, des éléments semblables sont dénotés par des références semblables dans les figures. La présence de numéros de référence aux dessins ne peut être considérée comme limitative, y compris lorsque ces numéros sont indiqués dans les revendications.

## Définitions

Un signal de mesure est un signal directement généré par un détecteur à infrarouge passif. Un signal de mesure peut être filtré et/ou amplifié.

Un signal discret de mesure est un signal de mesure qui est transmis à un appareil de traitement d'un signal selon une fréquence d'échantillonnage déterminée.

Un signal discret utile est un signal discret de mesure compris dans une fenêtre temporelle glissante dont la composante continue dudit signal discret de mesure a été soustraite.

Par détecteur à infrarouge passif, il faut comprendre un détecteur capable de détecter des ondes infrarouges issues d'un transfert de chaleur radiatif mais non émetteur de telles ondes. Cependant, un détecteur à infrarouge passif selon l'invention peut émettre des ondes telles que des ondes transmises selon un protocole WI-FI ou un protocole Zigbee pour la transmission de l'information relative aux ondes infrarouges détectées et mesurées. Un exemple de détecteur à infrarouge passif peut être un détecteur comprenant deux éléments pyroélectriques sensibles à un rayonnement infrarouge. Les deux éléments pyroélectriques reçoivent des ondes infrarouges et fournissent un signal proportionnel à la quantité d'ondes infrarouges reçues. Un différentiel de quantité d'ondes infrarouges reçues par chacun des éléments pyroélectriques d'un même détecteur à infrarouge passif est calculé par ledit détecteur, et ledit détecteur génère un signal électrique, le signal de mesure. Typiquement, c'est lorsqu'un mouvement d'un corps humain ou animal se produit dans le champ de détection d'un capteur à infrarouge passif qu'un différentiel existe entre les deux éléments pyroélectriques d'un même détecteur à infrarouge passif. Lors du déplacement d'un corps humain ou animal, les deux éléments pyroélectriques ne reçoivent pas la même quantité d'ondes infrarouges. Un détecteur à infrarouge passif transforme donc un mouvement en signal électrique.

## Description détaillée de modes de réalisation particuliers

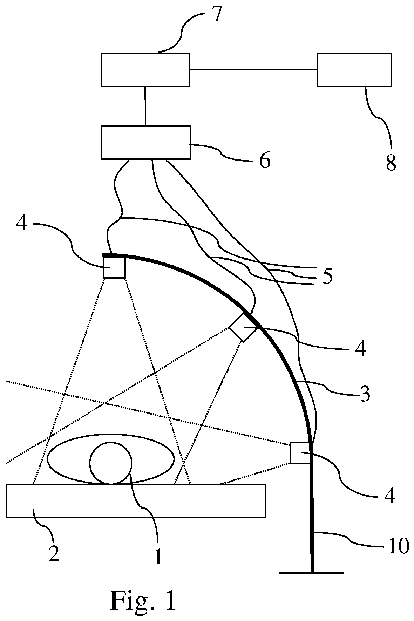

On se référera tout d'abord à la **fig. 1** qui représente un dispositif selon l'invention. Au moins une partie du dispositif peut être fixée sur un pied (9) à proximité d'un lit (2) où se trouve l'individu (1) à surveiller. Au moins une partie du dispositif peut également être disposée sur une structure surnuméraire et solidaire d'un lit (2) et /ou intégrée dans la structure propre du lit (2). Cette partie du dispositif comprend au moins un détecteur à infrarouge passif (4) ciblant au moins un endroit du patient susceptible de présenter des mouvements respiratoires, comme la cage thoracique ou l'abdomen par exemple. Le dispositif peut également comprendre un support courbe (3) destiné à supporter au moins un détecteur à infrarouge passif (4). De façon préférentielle, un détecteur à infrarouge passif (4) est un détecteur comprenant au moins 2 capteurs piézoélectriques (« dual sensor »), et de façon encore plus préférentielle au moins 4 capteurs piézoélectriques (« quad sensor »), un capteur piézoélectrique étant un élément sensible au rayonnement infrarouge passif. Selon ces modes de réalisation, la détection d'un mouvement respiratoire est encore plus sensible lorsque plusieurs éléments piézoélectriques sont présents De plus, lorsqu'un détecteur à infrarouge passif (4) comprend au moins 4 capteurs piézoélectrique, il est possible de réduire les problèmes liés à la sensibilité directionnelle des détecteurs. Le ou les détecteurs à infrarouge passifs (4) sont reliés par des moyens de connexion (5) à un amplificateur différentiel (6). L'amplificateur différentiel (6) est connecté à un moyen de traitement de signaux (7). Le moyen de traitement de signaux (7) est relié à un moyen d'alarme (8) visuel et/ou sonore situé à proximité ou à distance du dispositif.

Les mouvements respiratoires étant des mouvements d'une amplitude réduite, il est possible d'ajouter à l'au moins un détecteur à infrarouge passif (4) un moyen permettant de concentrer les ondes infrarouges incidentes vers les éléments pyroélectriques afin d'améliorer la sensibilité des détecteurs à infrarouge passifs (4). Un tel moyen peut par exemple être une lentille de Fresnel ou une lentille parabolique segmentée. Un tel moyen permet de concentrer le signal utile arrivant vers les éléments pyroélectriques d'un détecteur à infrarouge passif (4). Ces moyens sont bien connus de l'homme du métier et ne seront pas plus décrits. Alternativement, il est possible d'ajouter à l'au moins un détecteur à infrarouge passif (4) une lentille permettant de détecter les ondes présentes dans une zone centrale du détecteur et permettant concomitamment la détection d'ondes présentes en périphérie du détecteur. Une lentille à champ large, telle qu'une lentille permettant la détection d'ondes présentes dans un champ du détecteur à infrarouge passif compris entre -90° et +90° selon le champ horizontal, et -84° et +84° selon le champ vertical peut par exemple être utilisée. Une telle lentille permet d'éviter la présence d'un champ aveugle (absence de détection de toute variation d'onde infrarouge dans le champ aveugle) dans un champ de détection d'un détecteur passif à infrarouge (4).

Le détecteur à infrarouges passif (4) peut également être débridé afin d'avoir une sensibilité aux mouvements de faible amplitude accrue. Le détecteur (4) est ainsi plus sensible aux mouvements, et sa dynamique de détection est augmentée. La sensibilité du détecteur peut ainsi être comprise entre 0.1 e 10 $V_{pp}$, plus préférentiellement entre 3 et 4,5 $V_{pp}$, et de façon encore plus préférentielle, entre 3,3 et 3,6 $V_{pp}$.

Il n'y a pas de limitation au nombre de détecteurs à infrarouge passifs (4) que peut comprendre le dispositif. Ce nombre peut par exemple être de 1, 2, 3, 4, 5, 6, 7, 8, 9 ou 10 détecteurs. Le nombre de détecteurs peut être compris entre 1 et 10, entre 1 et 9, entre 1 et 8, entre 3 et 6. De manière préférentielle, le nombre de détecteurs est de 6, répartis en 3 boitiers comprenant chacun 2 détecteurs à infrarouge passifs (4). La distance entre les détecteurs à infrarouge passifs (4) et l'endroit du corps d'un individu ou d'un animal susceptible de présenter des mouvements périodiques liés à la respiration peut être comprise entre 20 cm et 200 cm, de préférence entre 25 cm et 150 cm. Le positionnement de détecteurs à infrarouge passifs (4) autour de l'endroit du corps d'un individu ou d'un animal susceptible de présenter des mouvements périodiques liés à la respiration peut se faire selon un arc de cercle (3) de 90° comme montré à la **fig. 1**. Par « *ciblant au moins un endroit du patient susceptible de présenter des mouvements respiratoires* », il faut comprendre que l'endroit du patient susceptible de présenter des mouvements respiratoires est dans le champ de détection de l'au moins un détecteur à infrarouge passif (4). Le champ de détection d'un détecteur à infrarouge passif (4) est généralement un cône avec une ouverture comprise entre 30° et 70°. Ce champ peut être modifié par l'ajout de lentilles comme expliqué précédemment.

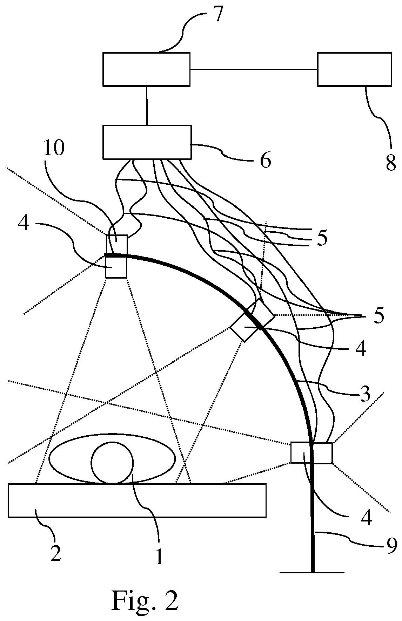

Les mouvements respiratoires étant des mouvements d'une amplitude réduite, le signal de mesure en sortie de l'au moins un détecteur à infrarouge passif peut être d'amplitude réduite. Il est possible d'utiliser un amplificateur différentiel (6) de signal pour détecteur à infrarouge passif. Cet amplificateur est relié à l'au moins un détecteur à infrarouge passif (4) par des moyens classiques de connexion (5) entre un amplificateur (6) et un détecteur à infrarouge passif (4). Chaque détecteur à infrarouge passif (4) est relié à l'amplificateur (6). L'amplificateur (6) est relié à un moyen de traitement (7) de signaux. Un convertisseur analogique/digital peut également être présent entre l'amplificateur (6) et le moyen de traitement (7) de signaux. Ces amplificateurs (6) sont bien connus de l'art antérieur. Un amplificateur différentiel (6) peut par exemple être un amplificateur opérationnel. Le moyen de traitement (7) de signaux est relié à une alarme visuelle et/ou sonore (8). Selon un mode de réalisation préféré de l'invention, comme montré à la **fig. 2**, un détecteur à infrarouge passif additionnel (10) ciblant l'environnement du patient (1), ou une partie du patient (1) non susceptible de présenter des mouvements liés à la respiration, est présent. Le moyen de traitement de signal peut être un moyen de traitement du signal analogique, et/ou numérique. De façon préférentielle, le moyen de traitement du signal est un moyen numérique.

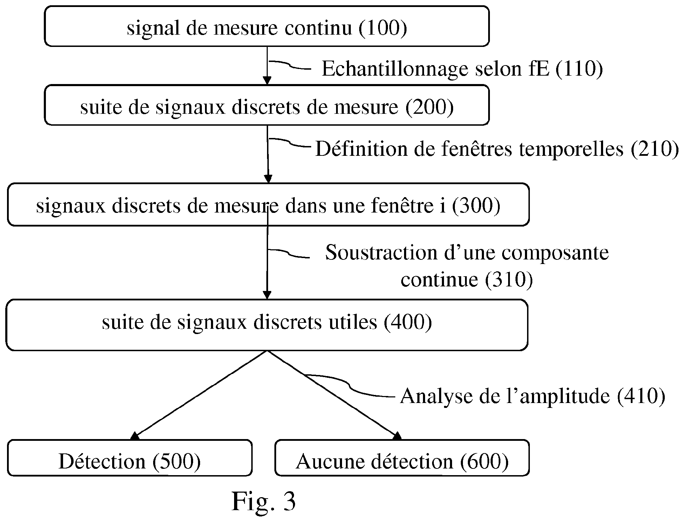

On se référera ensuite à la **fig. 3**. Le procédé de surveillance de la respiration d'un patient comprend les étapes consistant à :

i) fournir au moins un détecteur à infrarouge passif ciblant au moins un endroit du patient susceptible de présenter des mouvements respiratoires,

ii) fournir, selon une fréquence d'échantillonnage fE (110), au moins une suite de signaux discrets de mesure (200) ayant comme origine l'au moins un détecteur à infrarouge passif,

iii) définir une série de N fenêtres temporelles glissantes (210), débutant à des moments successifs différents $T_{in,i}$, i étant un entier compris entre 1 et N représentant chaque fenêtre temporelle glissante, de longueur $\Delta T_i = T_{f,i} - T_{in,i}$, $T_{f,i}$ désignant un temps final d'une fenêtre temporelle glissante numéro i,

iv) soustraire à chacun desdits signaux discrets de mesure présents dans une même fenêtre temporelle glissante (300) une composante continue (310) calculée sur une pluralité de ces signaux discrets de mesure (300) présents dans une même fenêtre temporelle glissante pour obtenir au moins une suite temporelle de signaux discrets utiles (400),

v) générer à chaque temps final $T_{f,i}$ un indicateur de mouvements respiratoires (410) calculé à partir d'une amplitude moyenne calculée sur un ensemble de signaux discrets utiles (400) compris dans une fenêtre temporelle glissante de longueur $\Delta T_i$ précédant ledit temps $T_{f,i}$ de la fenêtre temporelle glissante numéro i.

L'au moins un détecteur à infrarouge passif ciblant au moins un endroit du patient susceptible de présenter des mouvements respiratoires détecte les mouvements d'une source de chaleur de façon continue et transforme ce mouvement en un signal de mesure continu (100). Ce signal de mesure continu (100) est transmis par l'au moins un détecteur à infrarouge passif (4) à un amplificateur (6), ce dernier transmettant le signal amplifié au moyen de traitement de signaux (7). La transmission des signaux de mesure se fait selon une fréquence d'échantillonnage fE déterminée (110), transformant ainsi un signal de mesure continu (100) en une suite de signaux discrets de mesure (200). Cette étape est donc un échantillonnage d'un signal de mesure continu (100), permettant l'obtention d'une suite de signaux discrets de mesure (200), suite composée de plusieurs mesures du signal, relevées à plusieurs moments successifs, selon une fréquence d'échantillonnage fE déterminée (110). Cette fréquence fE peut par exemple être comprise entre 0.1 et 100 Hertz. Préférentiellement, cette fréquence peut être comprise entre 0.1 et 50 Hertz. Encore plus préférentiellement, cette fréquence peut être comprise entre 1 et 10 Hertz. De façon toujours plus préférentielle, la fréquence fE est de 10 Hertz. De façon préférentielle, il est possible de sélectionner une gamme de longueurs d'ondes transmises par l'au moins un détecteur à infrarouge passif (4) afin de ne transmettre qu'une partie des ondes infrarouges. Ainsi, il est possible de transmettre un signal de mesure continu (100) uniquement pour les longueurs d'ondes comprises entre 8 $\mu$m et 14 $\mu$m. Cette sélection de longueur d'ondes peut se réaliser grâce à un filtre optique, présent sur l'au moins détecteur à infrarouge passif (4), et/ou par la présence d'un filtre au niveau du capteur piézoélectrique de l'au moins un détecteur à infrarouge passif (4), ne permettant au capteur piézoélectrique de transmettre que les signaux correspondants à des variations de longueurs d'ondes comprises entre 8 $\mu$m et 14 $\mu$m. De façon toujours préférentielle, il est possible d'amplifier une gamme de fréquences particulières. Ainsi, il est préférable d'amplifier uniquement les signaux dont la fréquence est comprise entre 0.01 Hz et 10 Hz, et de façon encore plus préférentielle entre 0.01 Hz et 7 Hz. Selon ce mode de réalisation préférentiel, seuls les signaux présents dans la fenêtre de fréquence définie sont amplifiés, ces signaux représentant le signal utile car représentant la fréquence su mouvement respiratoire. Les signaux présents hors de cette fenêtre d'amplification ne sont donc pas amplifiés, ce qui permet d'améliorer l'analyse ultérieure du signal utile, car ces signaux représentent des phénomènes autres qu'un mouvement respiratoire. Il est possible de sélectionner les signaux ayant une longueur d'onde comprise entre 0.01 Hz et 10 Hz, plus préférentiellement entre 0.01 Hz et 7 Hz, par la présence d'un filtre passe-bas (permettant de ne garder que les signaux ayant une fréquence inférieure à la limite supérieure déterminée) et un filtre passe-bas (permettant de ne garder que les signaux ayant une fréquence supérieure à la limite inférieure déterminée). Les ondes ainsi sélectionnées sont ensuite amplifiées via un amplificateur opérationnel. Il est également possible de définir une fréquence de coupure au niveau de l'amplification du signal dans le but d'améliorer la résolution ultérieure du signal transmis. Cette opération a pour but de s'affranchir d'un bruit présent dans le signal transmis. Afin de s'affranchir de ce bruit, tout signal dont la fréquence est supérieure à une valeur prédéfinie n'est pas prise en compte lors de l'analyse ultérieure du signal.

Lorsque plus de un détecteur à infrarouge passif (4) ciblant au moins un endroit du patient (1) susceptible de présenter des mouvements respiratoires est présent, chacun des détecteurs à infrarouge passifs (4) ciblant au moins un endroit du patient (1) susceptible de présenter des mouvements respiratoires détecte les mouvements respiratoires de façon continue et transforme ce mouvement en un signal de mesure continu (100). Chacun des signaux de mesure continus (100) est transmis selon une même fréquence d'échantillonnage fE déterminée (110), transformant ainsi chacun des signaux de mesure continus (100) en autant de suites de signaux discrets de mesure (200). Chacune des suites de signaux discrets de mesure (200) est ensuite analysée séparément.

Les séries de signaux discrets de mesure (200) peuvent être traitées au fur et à mesure de leur transmission au moyen de traitement d'un signal (7) lors de la surveillance d'un individu (1), mais elles peuvent également être stockées en vue d'une analyse ultérieure.

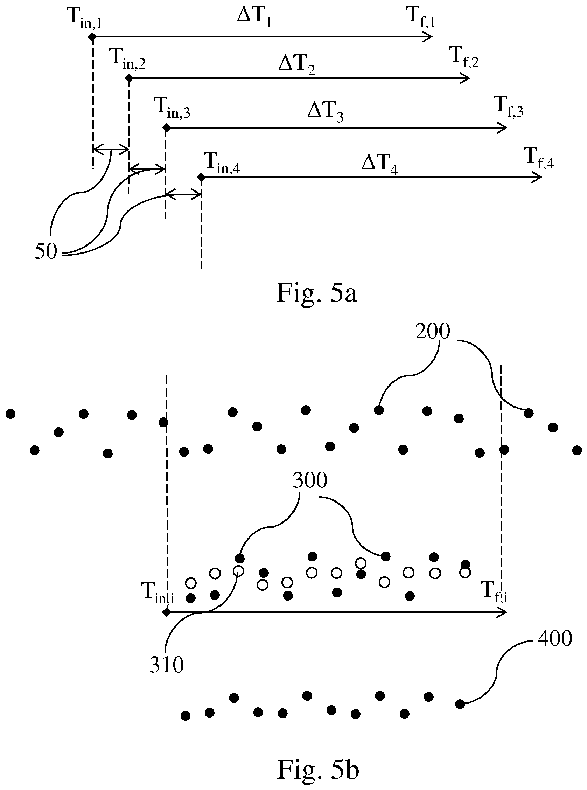

Le procédé comprend une étape consistant à définir une série de N fenêtres temporelles glissantes (210), débutant à des moments successifs différents $T_{in,i}$, i étant un entier compris entre 1 et N et représentant chaque fenêtre temporelle glissante, de longueur $\Delta T_i = T_{f,i} - T_{in,i}$, $T_{f,i}$ désignant un temps final d'une fenêtre temporelle glissante numéro i. N est un nombre entier supérieur à 0. Cette étape du procédé est illustrée à la **fig. 5a**. Une fenêtre glissante i est délimitée par des bornes ($T_{in,i}$ et $T_{f,i}$) glissantes dans le temps selon un pas de temps (50) prédéterminé. La longueur $\Delta T_i$ d'une fenêtre temporelle glissante peut être définie en fonction d'une durée d'une période respiratoire normale d'un patient. Cette fenêtre ne doit pas être inférieure à une durée d'une période respiratoire normale du patient, afin d'avoir une caractéristique périodique dans la série de signaux discrets de mesure (200). Cette fenêtre temporelle glissante ne doit pas non plus être trop longue, afin de détecter au plus tôt une absence de mouvement respiratoire. Cette fenêtre ne doit pas être inférieure à l'inverse de la fréquence d'échantillonnage fE ($\Delta T_i > 1/fE$). Par exemple, dans le cas d'un être humain adulte, si la durée d'une période respiratoire normale de l'individu est de 5 secondes, une fenêtre temporelle glissante peut être comprise entre 5 secondes et 20 secondes, de préférence entre 10 secondes et 15 secondes. Des fenêtres temporelles glissantes se succèdent selon un pas de temps déterminé. Un pas de temps peut par exemple être compris entre 0,5 seconde et 4 secondes, il peut également être compris entre 0,5 et 2 secondes. De façon préférentielle, un pas de temps entre chaque fenêtre temporelle est de 1 seconde. Chaque fenêtre temporelle glissante comprend donc un ensemble de signaux discrets de mesure (200) dont le nombre dépend de la durée de la longueur $\Delta T_i$ de la fenêtre temporelle glissante et de la fréquence d'échantillonnage fE. De façon préférentielle, les fenêtres temporelles glissantes ont une longueur $\Delta T_i$ constante, mais il est possible d'avoir des fenêtres temporelles glissantes de longueur $\Delta T_i$ variable. La méthode comprend donc une étape de détermination de fenêtres temporelles se succédant et pouvant se recouvrir afin d'obtenir un indicateur de mouvement respiratoire à chaque fenêtre temporelle, lesdites fenêtres temporelles pouvant se recouvrir, se succéder, et/ou être espacées dans le temps. Ainsi, les fenêtres temporelles définies permettent d'avoir un suivi continu de la respiration d'un patient ou d'un animal.

Le procédé comprend une étape consistant, pour chaque fenêtre temporelle glissante, à soustraire à chacun desdits signaux discrets de mesure présents dans une même fenêtre temporelle glissante (300) une composante continue (310) calculée sur une pluralité de ces signaux discrets de mesure présents dans une même fenêtre temporelle glissante (300) pour obtenir au moins une suite temporelle de signaux discrets utiles (400). Cette étape du procédé est illustrée à la **fig. 5b**. Une composante continue (310) d'un ensemble de signaux discrets de mesure d'une fenêtre considérée (300) peut par exemple être calculée en remplaçant chaque signal discret de mesure par une moyenne glissante calculée sur plusieurs signaux discrets de mesure de la même fenêtre temporelle (300). Par exemple, le nombre de signaux discrets de mesure à prendre en compte pour définir une composante continue peut être déterminé en fonction de la durée d'un rythme respiratoire normal de l'individu. Ainsi, dans le cas d'un être humain adulte avec une durée d'une période respiratoire normale de 5 secondes, le nombre de signaux discrets de mesure à considérer pour calculer une composante continue d'un ensemble de signaux discrets de mesure peut correspondre au nombre de signaux discrets de mesure nécessaires pour couvrir une durée d'environ 5 secondes. A l'extrémité des fenêtres temporelles glissantes, la moyenne glissante est calculée sur un nombre de signaux moins importants du fait du rapprochement des bornes de la fenêtre glissante. Une moyenne glissante peut aussi être calculée sur un nombre de signaux discrets de mesure (300) compris entre 15 et 45. Par exemple, lorsque la fréquence fE est égale à 10 Hz, une moyenne glissante peut être calculée avec 45 signaux discrets de mesure (300). La moyenne glissante ainsi calculée pour chaque signal discret est soustraite à chaque signal discret d'un ensemble de signaux discrets de mesure de la fenêtre temporelle glissante considéré (300) afin d'obtenir une suite temporelle de signaux discrets utiles (400).

Le procédé comprend également une étape consistant à générer à chaque temps final $T_{f,i}$ un indicateur de mouvements (410) respiratoires calculé à partir d'une amplitude moyenne calculée sur un ensemble de signaux discrets utiles (400) compris dans une fenêtre temporelle glissante de longueur $\Delta T_i$ précédant ledit temps $T_{f,i}$ de la fenêtre temporelle glissante numéro i. L'analyse est donc une analyse de signaux discrets et non continus. Le signal est donc analysé par la détermination de son amplitude, c'est-à-dire l'importance de la variation de sa fréquence, représentant l'amplitude du mouvement respiratoire. C'est l'importance du mouvement respiratoire qui est déterminante pour la détection ou non d'un mouvement respiratoire, et non sa fréquence. La fréquence de respiration est prise en compte lors de la définition des fenêtres temporelles glissantes. Le procédé comprend une étape d'analyse de l'amplitude du signal, et la fréquence de cette amplitude est indirectement prise en compte par la définition des fenêtres temporelles glissantes d'une durée déterminée et se suivant, et/ou se recouvrant selon un pas de temps déterminé, aboutissant à un procédé plus fin et plus complet. Ainsi, avec la méthode de l'invention, il est possible de détecter un mouvement respiratoire de faible amplitude et de le qualifier comme absence de mouvement respiratoire lorsque l'amplitude de ce mouvement est inférieure à un seuil prédéterminé. Il est ainsi possible de classer certains mouvements respiratoires de faible amplitude, souvent synonymes de l'existence d'un problème, comme représentant une absence de mouvement respiratoire. Ainsi, si un patient ou un animal présente un mouvement respiratoire de faible amplitude, une alerte peut être donnée.

En fonction de la valeur d'une amplitude moyenne d'un ensemble de signaux discrets utiles (400) compris dans une fenêtre temporelle glissante de longueur $\Delta T_i$, le procédé va classer ladite fenêtre temporelle considérée dans la catégorie de détection d'un mouvement respiratoire (500), ou dans la catégorie de l'absence de détection d'un mouvement respiratoire (600). Le seuil de détection d'un mouvement respiratoire par calcul de l'amplitude moyenne d'un ensemble de signaux discrets utiles compris dans une fenêtre temporelle glissante de longueur $\Delta T_i$ est dépendant du bruit généré par la chaine de mesure (détecteur à infrarouge passif, amplificateur) et peut donc être déterminé empiriquement. En fonction de la nature du détecteur à infrarouge passif et de l'amplificateur opérationnel associé, le seuil de détection de la présence d'un mouvement respiratoire peut être déterminé par exemple par la réalisation de la méthode dans des conditions contrôlées. Par exemple, en présence d'un détecteur à infrarouge passif Murata IRA-E700, d'un amplificateur OPA2340UA, ce seuil est compris entre 150 et 160 mV. Ainsi, le seuil de détermination de la détection d'un mouvement respiratoire par calcul d'une amplitude moyenne peut être fixé à 150mV, 151 mV, 152mV, 153mV, 154mV, 155mV, 156mV, 157mV, 158mV, 159mV, ou 160mV. Si la valeur de l'amplitude moyenne est supérieure ou égale à ce seuil, alors un mouvement respiratoire a été détecté (500).

De manière préférentielle, lors de l'étape consistant à générer à chaque pas de temps final $T_{f,i}$ un indicateur de mouvements respiratoires (410), cet indicateur est calculé à partir d'une amplitude moyenne calculée sur un ensemble de signaux discrets utiles (400) compris dans une fenêtre temporelle glissante de longueur $\Delta T_i$ précédant directement ledit temps $T_{f,i}$ de la fenêtre temporelle glissante numéro i.

Selon un mode de réalisation préféré de l'invention, le calcul de l'amplitude moyenne de l'étape v) du procédé comprend les étapes de :

- calcul d'une valeur d'amplitude RMS d'un ensemble de signaux discrets utiles (400) compris dans une fenêtre temporelle glissante de longueur $\Delta T_i$,
- comparaison de ladite valeur RMS avec un seuil prédéfini de détection d'un mouvement respiratoire par calcul d'une valeur RMS.

Par valeur d'amplitude RMS, il faut comprendre la valeur quadratique moyenne de l'amplitude, soit la racine de la somme des carrés divisée par le nombre d'éléments de la somme. L'étape de calcul d'une valeur d'amplitude RMS permet de mesurer la composante oscillante d'un ensemble de signaux discrets utiles (400) compris dans une fenêtre temporelle glissante considérée, et donc d'obtenir une amplitude moyenne du signal composé de l'ensemble des signaux discrets utiles (400) compris dans la fenêtre temporelle glissante considérée. Le seuil de détection d'un mouvement respiratoire par calcul d'une valeur RMS est dépendante du bruit généré par la chaine de mesure (détecteur à infrarouge passif, amplificateur) et peut donc être déterminé empiriquement. En fonction de la nature du détecteur à infrarouge passif et de l'amplificateur opérationnel associé, le seuil de détection de la présence d'un mouvement respiratoire peut être déterminé par exemple par la réalisation de la méthode dans des conditions contrôlées. Par exemple, en présence d'un détecteur à infrarouge passif Murata IRA-E700, d'un amplificateur OPA2340UA, ce seuil est compris entre 150 et 160 mV. Ainsi, le seuil de détermination de la détection d'un mouvement respiratoire par calcul d'une amplitude RMS peut être fixé à 150mV, 151 mV, 152mV, 153mV, 154mV, 155mV, 156mV, 157mV, 158mV, 159mV, ou 160mV. Si la valeur de l'amplitude RMS est supérieure ou égale à ce seuil, alors un mouvement respiratoire a été détecté (500).

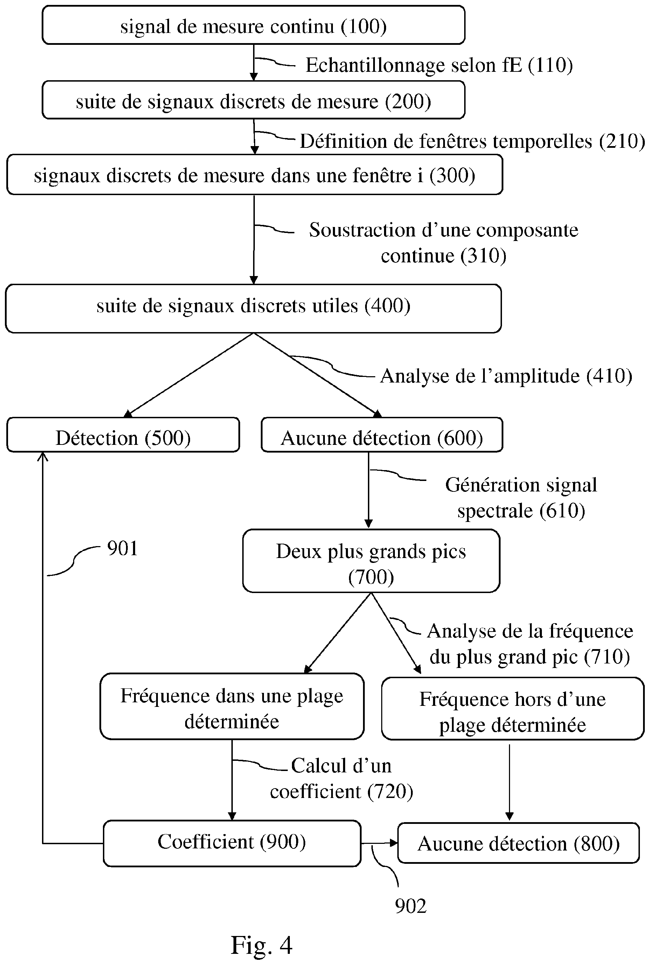

Selon un mode de réalisation préféré de l'invention illustré à la **fig. 4** une étape surnuméraire est présente. Cette étape consiste à :

- générer un signal spectral (610) d'un ensemble de signaux discrets utiles (400) compris dans une fenêtre temporelle glissante de longueur $\Delta T_i$,
- déterminer les deux plus grands pics dudit signal spectral (700),
- comparer (710) la fréquence du plus grand pic avec une plage de fréquence plausible des mouvements respiratoires,
- Calculer (720) un coefficient (900) selon la formule :

$$\frac{(H1 - H2)^2}{Hx}$$

où,

H1 est l'amplitude du pic le plus élevé,
H2 est l'amplitude du second pic le plus élevé,

Hx est l'amplitude du second pic le plus élevé lorsque cette amplitude est différente de zéro, ou l'amplitude du pic le plus élevé lorsque l'amplitude du second pic le plus élevé est égale à zéro,

- déterminer la présence d'un mouvement respiratoire par la comparaison entre le coefficient (900) obtenu à l'étape précédente et un seuil prédéterminé de détection d'un mouvement respiratoire par calcul d'un coefficient.

Cette étape surnuméraire est présente afin d'analyser un ensemble de signaux discrets utiles (400) compris dans une fenêtre temporelle glissante lorsque l'analyse de l'amplitude moyenne ne permet pas de détecter la présence d'un mouvement respiratoire (600), et donc que l'amplitude moyenne est inférieure au seuil de détection d'un mouvement respiratoire par calcul (410) de l'amplitude moyenne d'un ensemble de signaux discrets utiles (400) compris dans une fenêtre temporelle glissante. En effet, il arrive que le bruit de fond soit trop important pour que l'analyse de l'amplitude moyenne permette de donner un résultat exploitable pour certains ensembles de signaux discrets utiles compris dans une fenêtre temporelle glissante. Cette étape permet de distinguer encore mieux la présence d'un mouvement respiratoire et l'absence d'un mouvement respiratoire.

Ainsi, lorsqu'une amplitude moyenne d'un ensemble de signaux discrets utiles compris dans une fenêtre temporelle glissante est inférieure au seuil de détection d'un mouvement respiratoire par calcul de l'amplitude moyenne ou par calcul de la valeur RMS (600), une analyse spectrale (610) est réalisée sur ledit ensemble de signaux discrets utiles (400) compris dans une fenêtre temporelle glissante considérée. Cette analyse spectrale peut se faire à partir d'un nombre quelconque de signaux discrets utiles (400). Selon un mode de réalisation préféré de l'invention, une analyse spectrale peut se faire grâce à une transformée de Fourier, auquel cas un nombre de signaux égal à une puissance de deux peut être sélectionné parmi l'ensemble de signaux discrets utiles (400) compris dans une fenêtre temporelle glissante considérée. Cette sélection peut se faire de manière arbitraire.

La fréquence des deux plus grands pics dudit signal spectrale est déterminée. Si la fréquence du plus grand pic est en dehors d'une plage de fréquence déterminée (710), alors la fenêtre temporelle glissante considérée est analysée comme une absence de détection d'un mouvement respiratoire (800). Cette plage de fréquence déterminée (710) correspond à une plage de fréquence plausible pour détecter une respiration. Par exemple, pour un être humain adulte, la durée d'une période respiratoire, qui peut être variable en fonction de l'âge et de la physiologie de la personne, est comprise entre 1 seconde et 5 secondes. La plage de fréquence peut dans ce cas être comprise entre environ 0.20 Hz et environ 1 Hz ; plus préférentiellement entre 0.23 et 1.02 Hz. Si la fréquence du plus grand pic est comprise dans ladite plage de fréquence déterminée, alors un coefficient est calculé (720) selon la formule précédente. Si le résultat du coefficient (900) est supérieur ou égal (901) à une valeur déterminée, alors la fenêtre temporelle glissante considérée est analysée comme une présence d'un mouvement respiratoire (500). Si le résultat du coefficient (900) est inférieur (902) à une valeur déterminée, alors la fenêtre temporelle glissante considérée est analysée comme une absence d'un mouvement respiratoire (800). Ce coefficient (900) est déterminé de façon empirique de manière à éliminer les détections erronées sans éliminer les vraies détections d'un mouvement respiratoire. Ce coefficient (900) peut par exemple être égal à 10.

Selon un mode de réalisation préféré de l'invention, une étape consistant à :

- identifier pour chaque fenêtre temporelle glissante i si un ensemble de signaux discrets utiles (400) compris dans ladite fenêtre temporelle glissante indique une détection d'un mouvement respiratoire (500),
- lorsqu'aucune fenêtre temporelle glissante à un temps donné $T_{f,i}$ n'indique une détection de mouvement respiratoire (600 ou 800), classer ladite fenêtre temporelle glissante i comme étant dépourvue de mouvement respiratoire,
- lorsqu'un nombre déterminé de fenêtres temporelles glissantes successives sont classées comme étant dépourvues d'un mouvement respiratoire (600 ou 800), déclencher une alarme visuelle et/ou sonore,

est présente. Pour chacun des au moins un détecteur à infrarouge passif, chaque fenêtre temporelle glissante est analysée comme détectant la présence d'un mouvement respiratoire (500), ou l'absence d'un mouvement respiratoire (600 ou 800), à un temps donné $T_{f,i}$. Lorsqu'un un seul détecteur à infrarouge passif est présent, le résultat de l'analyse d'un ensemble de signaux discrets utiles (400) d'une fenêtre temporelle glissante correspond à la détection ou non d'un mouvement respiratoire. Lorsque au moins deux détecteurs à infrarouge passifs sont présents, chacune des fenêtres temporelles glissantes concomitantes à un temps donné $T_{f,i}$ issues de chacun de au moins deux détecteurs à infrarouge passifs est analysée, et le résultat le plus favorable est conservé pour l'ensemble des fenêtres temporelles glissantes concomitantes au temps donné $T_{f,i}$. Ainsi, si une seule fenêtre temporelle glissante est classée comme une détection de la présence d'un mouvement respiratoire (500), alors l'ensemble des fenêtres temporelles glissantes au temps donné $T_{f,i}$ est classé comme détectant la présence d'un mouvement respiratoire.

Lorsque toutes les fenêtres temporelles glissantes concomitantes à un temps donné $T_{f,i}$, ou lorsque la seule fenêtre temporelle glissante à un temps donné $T_{f,i}$, sont analysées comme signifiant une absence de mouvement respiratoire (600 ou 800), alors l'ensemble est classé comme étant une absence de mouvement respiratoire. Après un nombre déterminé de fenêtres glissantes successives, si tous les ensembles successifs de fenêtres temporelles glissantes concomitantes à un temps donné $T_{f,i}$ et à chaque pas de temps sont classés comme étant une absence de mouvement respiratoire (600 ou 800), alors une étape de déclenchement d'une alarme visuelle et/ou sonore est présente. Le nombre de fenêtres temporelles glissantes successives nécessaire avant le déclenchement de ladite alarme est déterminé en fonction de la durée du pas de temps entre chaque fenêtre d'analyse glissante, et du patient, afin de déclencher une alarme lorsque la durée de l'absence de mouvement respiratoire est considérée comme anormale. Par exemple, dans le cas d'un être humain adulte, et que le pas de temps entre chaque fenêtre temporelle glissante est de 1 seconde, le nombre de fenêtres temporelles glissantes successives peut être compris entre 15 et 30, de préférence 20. Une période de temps minimum avant de déclencher une alarme peut être déterminée en fonction de l'espèce (un être humain ou un animal). A titre d'exemple, lorsque les mouvements respiratoires d'un être humain adulte sont surveillés, cette période de temps peut être comprise entre 10 et 60 secondes, ou entre 10 et 45 secondes, ou entre 10 et 30 secondes, ou entre 20 et 30 secondes. Alternativement, cette période de temps peut être de 10, 15, 20, 25, 30, 35, 40, 45, 50, 55 ou 60 secondes. Ainsi, lorsqu'une période durant laquelle aucune fenêtre temporelle glissante ne détecte un mouvement est comprise entre 10 et 60 secondes, ou entre 10 et 45 secondes, ou entre 10 et 30 secondes, ou entre 20 et 30 secondes, une alarme est déclenchée. Cette période de temps peut être prédéterminée en fonction d'un rythme respiratoire normal d'un individu ou d'un animal. Dans ce cas, la période de temps peut être égale ou supérieure à 2 cycles de respiration normale d'un individu ou d'un animal.

Dans un mode de réalisation préféré de l'invention, une étape de traitement d'un signal discret de mesure (200) est présente. Cette étape consiste à appliquer un filtre médian et un filtre moyennant (ou passe-bas) sur lesdits signaux discrets de mesure (200). Cette étape permet d'éliminer des oscillations trop élevées du signal et des fréquences trop élevées, améliorant encore plus l'analyse ultérieure du signal discret utile (400). Par exemple, le filtre médian peut consister à remplacer chaque signal discret de mesure (200) par la valeur médiane de ce signal discret de mesure, du signal discret de mesure le précédant, et du signal discret de mesure le suivant. Chaque signal discret de mesure est donc transformé en un signal discret de mesure médian résultant de l'application d'un filtre médian sur plusieurs signaux successifs. De manière préférentielle, le nombre de signaux successifs utilisé pour calculer la valeur médiane est de 3, 5, 7, 9, 11, 13, 15, 17, 19 ou 21. De manière encore plus préférentielle, la valeur médiane est calculée à partir de 3 signaux successifs. Un filtre passe-bas peut par exemple consister à remplacer chaque signal discret de mesure (200) par une moyenne calculée sur la valeur de ce signal discret de mesure, du signal discret de mesure le précédant, et du signal discret de mesure le suivant.

Selon un mode de réalisation encore plus préféré de l'invention, illustré à la **fig. 2**, au moins un détecteur à infrarouge passif additionnel (10) ciblant une partie du corps non susceptible de présenter des mouvements respiratoires ou ciblant l'environnement d'un individu est fourni. Cet au moins un détecteur à infrarouge passif additionnel (10) est utilisé pour fournir, selon une fréquence d'échantillonnage fE, une suite de signaux discrets de mesure du bruit de fond, appelé signal de fond, ayant comme origine ledit détecteur additionnel (10). Ce détecteur peut être identique à l'au moins un détecteur à infrarouge passif ciblant une partie du corps susceptible de présenter des mouvements respiratoires.

L'au moins un détecteur à infrarouge passif additionnel (10) ciblant une partie du corps non susceptible de présenter des mouvements respiratoires ou ciblant l'environnement d'un individu peut par exemple être dirigé de façon substantiellement perpendiculaire à l'au moins un détecteur à infrarouge passif (4) ciblant une partie du corps susceptible de présenter des mouvements respiratoires. Le champ de détection du détecteur à infrarouge passif additionnel (10) ciblant une partie du corps non susceptible de présenter des mouvements respiratoires ou ciblant l'environnement d'un individu peut avoir un champ de détection compris entre 30 et 45°. Autrement dit, le champ de détection de l'au moins un détecteur à infrarouge passif additionnel (10) ciblant une partie du corps non susceptible de présenter des mouvements respiratoires ou ciblant l'environnement d'un individu n'est pas dirigé vers un endroit du patient susceptible de présenter des mouvements respiratoires. La sensibilité de l'au moins un détecteur à infrarouge passif ciblant une partie du corps non susceptible de présenter des mouvements respiratoires ou ciblant l'environnement d'un individu peut avoir une sensibilité équivalente à l'au moins un détecteur à infrarouge passif (4) ciblant une partie du corps susceptible de présenter des mouvements respiratoires.

Dans ce mode de réalisation préféré, une étape comprenant la soustraction d'un signal de fond ayant comme origine le détecteur à infrarouge passif additionnel (10) ciblant une partie du corps non susceptible de présenter des mouvements respiratoires ou ciblant l'environnement d'un individu à un signal discret de mesure (200) concomitant ayant comme origine le détecteur à infrarouge passif ciblant une partie du corps susceptible de présenter des mouvements respiratoires peut être présente. Le signal discret de mesure (200) ainsi généré est ensuite analysé selon les différentes étapes du procédé déjà décrit précédemment.

L'invention se rapporte aussi à un dispositif de surveillance de la respiration d'un individu ou d'un animal. Ce dispositif, illustré à la **fig. 1**, comprend :

- au moins un détecteur à infrarouge passif (4) apte à cibler au moins un endroit d'un patient (1) susceptible de présenter des mouvements respiratoires,
- au moins un amplificateur (6) pour l'au moins un détecteur à infrarouge passif (4),
- au moins un moyen de traitement (7) de signaux programmé pour réaliser une étape de définition d'une série de N fenêtres temporelles glissantes débutant à des moments successifs différents $T_{in,i}$ et de longueur $\Delta_{Ti}$ ; une étape de soustraction à chacun desdits signaux discrets de mesure présents dans une même fenêtre temporelle glissante d'une composante continue calculée sur une pluralité de ces signaux discrets de mesure présents dans une même fenêtre temporelle glissante pour obtenir une suite temporelle de signaux discrets utiles ; et une étape de génération à chaque temps final $T_{f,i}$ d'un indicateur de mouvements respiratoires calculé à partir d'une amplitude moyenne d'un ensemble de signaux discrets utiles compris dans une fenêtre glissante de longueur $\Delta T_i$,
- au moins une alarme visuelle et/ou sonore (8).

Ces éléments sont reliés entre eux par des moyens de connexion (5) classiques connus de l'homme du métier. Le dispositif peut également comprendre un convertisseur analogique/digital.

Le moyen de traitement (7) de signaux discrets est relié à un dispositif d'alarme (8) lumineux et/ou sonore, local ou à distance. Ce dispositif d'alarme (8) est déclenché par le moyen de traitement de signaux (7) lorsque ce dernier ne détecte pas de mouvements respiratoires durant une période de temps déterminée selon le procédé.

Selon un mode de réalisation préféré de l'invention, le dispositif comprend un moyen de traitement (7) des signaux discrets programmé pour réaliser les autres étapes du procédé. Ainsi le moyen de traitement (7) de signaux peut être programmé pour réaliser une étape de soustraction à un signal discret de mesure d'un signal de fond. Le moyen de traitement de signaux peut être programmé pour calculer une valeur d'amplitude RMS d'un ensemble de signaux discrets utiles, et comparer la dite valeur d'amplitude RMS avec un seuil de détection par calcul d'une amplitude moyenne. Le moyen de traitement de signal peut également être programmé pour générer un signal spectral d'un ensemble de signaux discrets utiles, déterminer la fréquence des deux plus grands pics présents dans ledit signal spectral, comparer la fréquence du plus grand pic avec une plage de fréquence plausible de mouvements respiratoires prédéterminée, calculer un coefficient de comparaison d'amplitude selon la formule :

$$\frac{(H1 - H2)^2}{Hx}$$

où,

H1 est l'amplitude du pic le plus élevé,
H2 est l'amplitude du second pic le plus élevé,
Hx est l'amplitude du second pic le plus élevé lorsque cette amplitude est différente de zéro, ou l'amplitude du pic le plus élevé lorsque l'amplitude du second pic le plus élevé est égale à zéro,

et comparer le coefficient de comparaison d'amplitude avec un seuil prédéterminé de détection par calcul d'un coefficient.

Le dispositif peut également être programmé pour classer un ensemble d'au moins une fenêtre glissante temporelle comme étant dépourvue de mouvement respiratoire et déclencher une alarme lorsqu'un nombre déterminé de fenêtres temporelles glissantes successives ne détectent pas de mouvements respiratoires.

Selon un mode de réalisation encore plus préféré de l'invention, illustré à la **fig. 2**, le dispositif comprend également au moins un détecteur à infrarouge passif additionnel (10) ciblant une partie du corps non susceptible de présenter des mouvements respiratoires ou ciblant l'environnement d'un patient.

Un exemple de réalisation de certaines étapes de la méthode est présenté en figure 5a et 5b. Plusieurs fenêtres temporelles ($\Delta T_1$, $\Delta T_2$, $\Delta T_3$, $\Delta T_4$) se succèdent selon un pas de temps (50) déterminé. Une suite de signaux discrets de mesures (200) est générée par un détecteur à infrarouge passif. Pour la fenêtre considérée i, seuls les signaux discrets de mesure compris dans l'intervalle de temps $\Delta T_i$ sont analysés. Pour chacun de ces signaux discrets de mesure compris dans la fenêtre $\Delta T_i$, une composante continue (310) est calculée en effectuant un calcul de la moyenne de chaque signal discret, du signal discret le précédent, et du signal discret le suivant. Cette composante continue (310) est soustraite à chaque signal discret de mesure (300), permettant l'obtention d'une suite temporelle de signaux discrets utiles (400). Pour cette suite (400), l'amplitude moyenne est calculée. La valeur de cette amplitude moyenne est ensuite comparée à un seuil de détection par calcul d'une amplitude moyenne prédéfini.

L'invention peut également se définir comme se rapportant à une méthode de détermination de la présence d'un mouvement respiratoire chez un être humain ou animal. Cette méthode comprend la détection de mouvement par un détecteur à infrarouge passif, et l'analyse de signaux transmis par ce détecteur. L'invention se rapporte également à un appareil pour mettre en oeuvre une méthode de détection d'un mouvement respiratoire.

**Revendications**

1. Procédé de surveillance de la respiration d'un patient comprenant les étapes suivantes :

i) fournir au moins un détecteur à infrarouge passif ciblant au moins un endroit du patient susceptible de présenter des mouvements respiratoires,

ii) fournir, selon une fréquence d'échantillonnage fE (110), au moins une suite de signaux discrets de mesure (200) ayant comme origine l'au moins un détecteur à infrarouge passif,

**caractérisé en ce que** le procédé comprend en outre les étapes suivantes :

iii) définir une série de N fenêtres temporelles glissantes (210), débutant à des moments successifs différents $T_{in,i}$, i étant un entier compris entre 1 et N représentant chaque fenêtre temporelle glissante, de longueur $\Delta T_i = T_{f,i} - T_{in,i}$, $T_{f,i}$ désignant un temps final d'une fenêtre temporelle glissante numéro i,

iv) soustraire à chacun desdits signaux discrets de mesure présents dans une même fenêtre temporelle glissante (300) une composante continue calculée sur une pluralité de ces signaux discrets de mesure (300) présents dans une même fenêtre temporelle glissante pour obtenir au moins une suite temporelle de signaux discrets utiles (400),

v) générer à chaque temps final $T_{f,i}$ un indicateur de mouvements respiratoires (410) calculé à partir d'une amplitude moyenne calculée sur un ensemble de signaux discrets utiles compris dans une fenêtre temporelle glissante de longueur $\Delta T_i$ précédant ledit temps $T_{f,i}$ de la fenêtre temporelle glissante numéro i.

2. Procédé selon la revendication 1 comportant en outre une étape consistant à:

- fournir au moins un détecteur à infrarouge passif additionnel ciblant au moins un endroit du patient non susceptible de présenter des mouvements respiratoires ou ciblant l'environnement du patient,
- soustraire aux signaux discrets de mesures (200) de l'étape ii) un signal de fond ayant comme origine l'au moins un détecteur à infrarouge passif ciblant au moins un endroit du patient non susceptible de présenter des mouvements respiratoires ou ciblant l'environnement du patient.

3. Procédé selon la revendication 1 ou 2, comprenant en outre une étape de calcul d'une amplitude moyenne comprenant les étapes consistant à:

- calculer une valeur d'amplitude RMS d'un ensemble des signaux discrets utiles (400) compris dans une fenêtre temporelle glissante de longueur $\Delta T_i$,

- comparer ladite valeur d'amplitude RMS avec un seuil prédéfini de détection d'un mouvement respiratoire par calcul d'une amplitude moyenne.

4. Procédé selon la revendication 3 comprenant en outre une étape consistant à :

- générer un signal spectral (610) d'un ensemble des signaux discrets utiles (400) compris dans une fenêtre temporelle glissante de longueur $\Delta T_i$,
- déterminer les deux plus grands pics dudit signal spectral (700),
- comparer (710) la fréquence du plus grand pic avec une plage de fréquence plausible des mouvements respiratoires,
- calculer (720) un coefficient (900) selon la formule :

$$\frac{(H1 - H2)^2}{Hx}$$

où,

H1 est l'amplitude du pic le plus élevé,
H2 est l'amplitude du second pic le plus élevé,
Hx est l'amplitude du second pic le plus élevé lorsque ce dernier est différent de zéro, ou l'amplitude du pic le plus élevé lorsque le second pic le plus élevé est égal à zéro,

- déterminer la présence d'un mouvement respiratoire par une comparaison entre le coefficient (900) obtenu à l'étape précédente et un seuil prédéterminé de détection d'un mouvement respiratoire par calcul d'un coefficient.

5. Procédé selon l'une quelconque des revendications précédentes, comprenant en outre une étape consistant à :

- identifier pour une fenêtre temporelle glissante si ledit ensemble de signaux discrets utiles (400) compris dans ladite fenêtre temporelle glissante indique une détection d'un mouvement respiratoire (500),
- si une fenêtre temporelle glissante indique une absence de détection d'un mouvement respiratoire (600 ou 800), classer ladite fenêtre temporelle comme étant dépourvue de mouvement respiratoire,
- si un nombre prédéterminé de fenêtres temporelles glissantes successives sont classées comme étant dépourvues d'un mouvement respiratoire (600 ou 800), déclencher une alarme visuelle et/ou sonore.

6. Procédé selon l'une quelconque des revendications précédentes **caractérisé en ce que** la fréquence d'échantillonnage fE est comprise entre 0,1 Hz et 50 Hz.

7. Procédé selon l'une quelconque des revendications précédentes **caractérisé en ce qu'**un filtre médian et un filtre passe-bas sont appliqués sur ladite suite temporelle de signaux discrets de mesure (200) de l'étape ii).

8. Procédé selon l'une quelconque des revendications précédentes **caractérisé en ce qu'**une fenêtre temporelle glissante a une longueur $\Delta T_i$ comprise entre 5 et 20 secondes.

9. Procédé selon les revendications 3 à 7 **caractérisé en ce que** le seuil de détection d'un mouvement respiratoire par calcul d'une amplitude moyenne d'un ensemble de signaux discrets utiles (400) compris dans une fenêtre temporelle glissante de longueur $\Delta T_i$ est compris entre 150mV et 160 mV.

10. Procédé selon les revendications 4 à 9 **caractérisé en ce que** le seuil de détection d'un mouvement respiratoire par un coefficient est supérieur ou égal à 10.

11. Dispositif de surveillance de la respiration d'un individu ou d'un animal comprenant :

   - au moins un détecteur à infrarouge passif (4) apte à cibler au moins un endroit du patient (1) susceptible de présenter des mouvements respiratoires,
   - au moins un amplificateur (6) pour l'au moins un détecteur à infrarouge passif (4),
   - au moins une alarme (8) visuelle et/ou sonore,

   **caractérisé en ce qu'**au moins un moyen de traitement (7) de signaux est programmé pour réaliser les étapes iii), iv) et v) de la revendication 1.

12. Dispositif selon la revendication précédente, **caractérisé en ce que** le moyen de traitement (7) de signaux est également programmé pour réaliser une étape de l'une quelconque des revendications 2, 3, 4, 5,7 ou 8.

13. Dispositif selon la revendication 11 ou 12, **caractérisé en ce qu'**il comporte au moins un détecteur à infrarouge passif additionnel (10) apte à cibler une partie du corps non susceptible de présenter des mouvements respiratoires ou apte à cibler l'environnement d'un individu.

**Patentansprüche**

1. Überwachungsverfahren der Atmung eines Patienten, umfassend die folgenden Schritte:

   i) Lieferung wenigstens eines passiven Infrarotdetektors, der wenigstens eine Stelle des Patienten anvisiert, die geeignet ist, Atembewegungen aufzuweisen,
   ii) je nach einer Probennahmenfrequenz fE (110) Lieferung wenigstens einer Folge von diskreten Messsignalen (200), deren Ursprung wenigstens ein passiver Infrarotdetektor ist,

   **dadurch gekennzeichnet, dass** das Verfahren darüber hinaus die folgenden Schritte umfasst:

   iii) Definition einer Serie von N gleitenden Zeitfenstern (210), die zu sukzessiven unterschiedlichen Momenten $T_{in, i}$ beginnen, wobei i eine zwischen 1 und N inbegriffene ganze Zahl ist, die jedes gleitende Zeitfenster, mit einer Länge von $\Delta T_i = T_{fi} - T_{in, i}$ darstellt, wobei $T_{f, i}$ eine Endzeit eines gleitenden Zeitfensters Nummer i bezeichnet,
   iv) jedem der genannten diskreten Messsignale, die in einem und demselben gleitenden Zeitfenster (300) vorhanden sind, eine kontinuierliche Komponente entziehen, die auf eine Vielzahl dieser diskreten Messsignale (300) berechnet ist, die in einem und demselben gleitenden Zeitfenster vorhanden sind, um wenigstens eine zeitliche Folge von nützlichen diskreten Signalen (400) zu erhalten,
   v) bei jeder Endzeit $T_{f, i}$ einen Atembewegungsindikator (410) generieren, der ausgehend von einer mittleren Amplitude berechnet ist, die auf eine Gruppe von nützlichen diskreten Signalen berechnet ist, welche in einem gleitenden Zeitfenster mit einer Länge $\Delta T_i$ inbegriffen sind, das der genannten Zeit $T_{f, i}$ des gleitenden Zeitfensters Nummer i vorausgeht.

2. Verfahren gemäß Anspruch 1, umfassend darüber hinaus einen Schritt, umfassend:

   - Lieferung wenigstens eines zusätzlichen passiven Infrarotdetektors, der wenigstens eine Stelle des Patienten anvisiert, die nicht geeignet ist, Atembewegungen aufzuweisen, oder die Umgebung des Patienten anvisiert,
   - den diskreten Messsignalen (200) des Schritts ii) ein Hintergrundsignal entziehen, dessen Ursprung wenigstens ein passiver Infrarotdetektor ist, der wenigstens eine Stelle des Patienten anvisiert, die nicht geeignet ist, Atembewegungen aufzuweisen, oder der die Umgebung des Patienten anvisiert.

**3.** Verfahren gemäß Anspruch 1 oder 2, umfassend darüber hinaus einen Berechnungsschritt einer mittleren Amplitude, die die Schritte umfasst, bestehend aus:

- Berechnung eines Amplitudenwertes RMS einer Gruppe von nützlichen diskreten Signalen (400), die in einem gleitenden Zeitfenster mit einer Länge $\Delta T_i$ inbegriffen sind,
- Vergleichen des genannten Amplitudenwertes RMS mit einem vorbestimmten Erfassungsschwellenwert einer Atembewegung per Berechnung einer mittleren Amplitude.

**4.** Verfahren gemäß Anspruch 3, umfassend darüber hinaus einen Schritt, bestehend aus:

- Generieren eines spektralen Signals (610) einer Gruppe der nützlichen diskreten Signale (400), die in einem gleitenden Zeitfenster mit einer Länge $\Delta T_i$ inbegriffen sind,
- Bestimmen der zwei größten Spitzen des genannten spektralen Signals (700),
- Vergleichen (710) der Frequenz der größten Spitze mit einem plausiblen Frequenzbereich der Atembewegungen,
- Berechnen (720) eines Koeffizienten (900) gemäß der Formel:

$$\frac{(H1 \ - \ H2)^2}{Hx}$$

bei der

H1 die Amplitude der höchsten Spitze ist,
H2 die Amplitude der zweithöchsten Spitze ist,
Hx die Amplitude der zweithöchsten Spitze ist, wenn diese unterschiedlich von Null ist, oder die Amplitude der höchsten Spitze ist, wenn die zweithöchste Spitze gleich Null ist,

- Bestimmung des Vorhandenseins einer Atembewegung durch einen Vergleich zwischen dem Koeffizienten (900), der in dem voranstehenden Schritt erhalten wird, und einem vorbestimmten Erfassungsschwellenwert einer Atembewegung per Berechnung eines Koeffizienten.

**5.** Verfahren gemäß irgendeinem der voranstehenden Ansprüche, umfassend darüber hinaus einen Schritt, bestehend aus:

- für ein gleitendes Zeitfenster identifizieren, ob die genannte Gruppe von nützlichen diskreten Signalen (400), die in dem genannten gleitenden Zeitfenster inbegriffen ist, eine Erfassung einer Atembewegung (500) angibt,
- wenn ein gleitendes Zeitfenster ein Fehlen einer Erfassung einer Atembewegung (600 oder 800) angibt, das genannte Zeitfenster als ohne Atembewegung einstufen,
- wenn eine vorbestimmte Anzahl von sukzessiven gleitenden Zeitfenstern als ohne Atembewegung (600 oder 800) eingestuft ist, einen visuellen und / oder Tonalarm auslösen.

**6.** Verfahren gemäß irgendeinem der voranstehenden Ansprüche, **dadurch gekennzeichnet, dass** die Probennahmenfrequenz fE zwischen 0,1 Hz und 50 Hz inbegriffen ist.

**7.** Verfahren gemäß irgendeinem der voranstehenden Ansprüche, **dadurch gekennzeichnet, dass** ein medianer Filter und ein Tiefpassfilter auf die genannte zeitliche Folge von diskreten Messsignalen (200) des Schritts ii) angewendet werden.

**8.** Verfahren gemäß irgendeinem der voranstehenden Ansprüche, **dadurch gekennzeichnet, dass** ein gleitendes Zeitfenster eine Länge $\Delta T_i$ hat, die zwischen 5 und 20 Sekunden inbegriffen ist.

**9.** Verfahren gemäß Anspruch 3 bis 7, **dadurch gekennzeichnet, dass** der Erfassungsschwellenwert einer Atembewegung per Berechnung einer mittleren Amplitude einer Gruppe von nützlichen diskreten Signalen (400), die in einem gleitenden Zeitfenster mit einer Länge $\Delta T_i$ inbegriffen ist, zwischen 150 mV und 160 mV inbegriffen ist.

**10.** Verfahren gemäß Anspruch 4 bis 9, **dadurch gekennzeichnet, dass** der Erfassungsschwellenwert einer Atembewegung durch einen Koeffizienten größer als oder gleich 10 ist.

**11.** Überwachungsverfahren der Atmung eines Individuums oder eines Tiers, umfassend:

- wenigstens einen passiven Infrarotdetektor (4), der geeignet ist, wenigstens eine Stelle des Patienten (1) anzuzielen, die geeignet ist, Atembewegungen aufzuweisen,
- wenigstens einen Verstärker (6) für wenigstens einen passiven Infrarotdetektor (4),
- wenigstens einen visuellen und / oder Tonalarm (8),

**dadurch gekennzeichnet, dass** wenigstens ein Verarbeitungsmittel (7) von Signalen programmiert ist, um die Schritte iii), iv) und v) des Anspruchs 1 zu realisieren.

**12.** Vorrichtung gemäß dem voranstehenden Anspruch, **dadurch gekennzeichnet, dass** das Verarbeitungsmittel (7) von Signalen ebenfalls programmiert ist, um einen Schritt gemäß irgendeinem der Ansprüche 2, 3, 4, 5, 7 oder 8 zu realisieren.

13. Vorrichtung gemäß Anspruch 11 oder 12, **dadurch gekennzeichnet, dass** sie wenigstens einen zusätzlichen passiven Infrarotdetektor (10) umfasst, der geeignet ist, einen Teil des Körpers anzuvisieren, der nicht geeignet ist, Atembewegungen aufzuweisen oder geeignet ist, die Umgebung eines Individuums anzuvisieren.

**Claims**

1. A method for monitoring the respiration of a patient, comprising the following steps:

   i) providing at least one passive infrared detector targeting at least one location on the patient capable of exhibiting respiratory movements,
   ii) providing, at a sampling frequency fE (110), at least one sequence of discrete measurement signals (200) originating from the at least one passive infrared detector,

   **characterized in that** the method further comprises the steps of:

   iii) defining a series of N sliding time windows (210), starting at different successive moments $T_{in,i}$, where i is an integer from 1 to N representing each sliding time window with a length of $\Delta T_i = T_{f,i} - T_{in,i}$, where $T_{f,i}$ denotes a final time of a sliding time window numbered i,
   iv) subtracting from each of said discrete measurement signals present in the same sliding time window (300) a continuous component calculated over a plurality of these discrete measurement signals (300) present in the same sliding time window, to obtain at least one temporal sequence of useful discrete signals (400),
   v) generating at each final time $T_{f,i}$ an indicator of respiratory movements (410), calculated on the basis of a mean amplitude calculated over a set of useful discrete signals included in a sliding time window with a length of $\Delta T_i$ preceding said time $T_{f,i}$ of the sliding time window numbered i.

2. The method as claimed in claim 1, further comprising a step consisting in:

   - providing at least one additional passive infrared detector targeting at least one location on the patient not capable of exhibiting respiratory movements, or targeting the patient's environment,

   - subtracting from the discrete measurement signals (200) of step ii) a background signal originating from the at least one passive infrared detector targeting at least one location on the patient not capable of exhibiting respiratory movements or targeting the patient's environment.

3. The method as claimed in claim 1 or 2, further comprising a step of calculating a mean amplitude comprising the steps consisting of:

   - calculating an RMS amplitude value of a set of useful discrete signals (400) included in a sliding time window with a length of $\Delta T_i$,
   - comparing said RMS amplitude value with a predefined threshold of detection of a respiratory movement by calculation of a mean amplitude.

4. The method as claimed in claim 3, further comprising a step consisting in:

   - generating a spectral signal (610) of a set of useful discrete signals (400) included in a sliding time window with a length of $\Delta T_i$,
   - determining the two highest peaks of said spectral signal (700),
   - comparing (710) the frequency of the highest peak with a plausible frequency range of the respiratory movements,
   - calculating (720) a coefficient (900) according to the formula:

   $$\frac{(H1 - H2)^2}{Hx}$$

   where

   H1 is the amplitude of the highest peak,
   H2 is the amplitude of the second highest peak,
   Hx is the amplitude of the second highest peak if this amplitude is other than zero, or the amplitude of the highest peak if the amplitude of the second highest peak is zero,

   - determining the presence of a respiratory movement by a comparison between the coefficient (900) obtained in the preceding step and a predetermined threshold of detection of a respiratory movement by calculation of a coefficient.

5. The method as claimed in any one of the preceding claims, further comprising a step consisting in:

- determining, for a sliding time window, whether said set of useful discrete signals (400) included in said sliding time window indicates the detection of a respiratory movement (500),
- if no sliding time window indicates an absence of detection of a respiratory movement (600 or 800), classifying said sliding time window as containing no respiratory movement,
- if a predetermined number of successive sliding time windows are classified as containing no respiratory movement (600 or 800), triggering a visual and/or audible alarm.

6. The method as claimed in any one of the preceding claims, **characterized in that** the sampling frequency fE is in the range from 0.1 Hz to 50 Hz.

7. The method as claimed in any one of the preceding claims, **characterized in that** a median filter and a low-pass filter are applied to said temporal sequence of discrete measurement signals (200) of step ii).

8. The method as claimed in any one of the preceding claims, **characterized in that** a sliding time window has a length $\Delta T_i$ in the range from 5 to 20 seconds.

9. The method as claimed in claims 3 to 7, **characterized in that** the threshold of detection of a respiratory movement by calculation of a mean amplitude of a set of useful discrete signals (400) included in a sliding time window with a length of $\Delta T_i$ is in the range from 150 mV to 160 mV.

10. The method as claimed in claims 4 to 9, **characterized in that** the threshold of detection of a respiratory movement by a coefficient is greater than or equal to 10.

11. A device for monitoring the respiration of a person or an animal, comprising:

- at least one passive infrared detector (4) adapted to target at least one location on a patient (1) capable of exhibiting respiratory movements,
- at least one amplifier (6) for the at least one passive infrared detector (4),
- at least one visual and/or audible alarm (8),

**characterized in that** at least one signal processing means (7) is programmed to execute steps iii), iv) and v) of claim 1,

12. The device as claimed in the preceding claim, **characterized in that** the signal processing means (7) is also programmed to execute a step of any one of claims 2, 3, 4, 5, 7 or 8.

13. The device as claimed in claim 11 or 12, **character-**

**ized in that** it includes at least one additional passive infrared detector (10), adapted to target a part of the body not capable of exhibiting respiratory movements or adapted to target the environment of a person.

Fig. 1

Fig. 2

signal de mesure continu (100)

└── Echantillonnage selon fE (110)

suite de signaux discrets de mesure (200)

└── Définition de fenêtres temporelles (210)

signaux discrets de mesure dans une fenêtre i (300)

└── Soustraction d'une composante
continue (310)

suite de signaux discrets utiles (400)

└── Analyse de l'amplitude (410)

Détection (500)      Aucune détection (600)

Fig. 3

signal de mesure continu (100)

⌐⌐— Echantillonnage selon fE (110)

suite de signaux discrets de mesure (200)

⌐⌐—Définition de fenêtres temporelles (210)

signaux discrets de mesure dans une fenêtre i (300)

⌐⌐— Soustraction d'une composante
continue (310)

suite de signaux discrets utiles (400)

⌐⌐—Analyse de l'amplitude (410)

Détection (500)      Aucune détection (600)

⌐⌐— Génération signal
spectrale (610)

⌐ 901

Deux plus grands pics
(700)

⌐⌐—Analyse de la fréquence
du plus grand pic (710)

Fréquence dans une plage
déterminée            Fréquence hors d'une
plage déterminée

⌐⌐—Calcul d'un
coefficient (720)

Coefficient (900)      Aucune détection (800)

902

Fig. 4

$$T_{in,1} \qquad\qquad \Delta T_1 \qquad\qquad T_{f,1}$$

$$T_{in,2} \qquad\qquad \Delta T_2 \qquad\qquad T_{f,2}$$

$$T_{in,3} \qquad\qquad \Delta T_3 \qquad\qquad T_{f,3}$$

$$T_{in,4} \qquad\qquad \Delta T_4 \qquad\qquad T_{f,4}$$

50

## Fig. 5a

200

300

$$T_{in,i} \qquad\qquad\qquad\qquad\qquad T_{f,i}$$

310

400

## Fig. 5b

## RÉFÉRENCES CITÉES DANS LA DESCRIPTION

**Documents brevets cités dans la description**

- US 20100331632 A
- WO 2005020815 A